# EUROPÄISCHE PATENTSCHRIFT

(11) **EP 0 898 168 B1**
(45) Veröffentlichungstag und Bekanntmachung des Hinweises auf die Patenterteilung: **06.10.2004**
(21) Anmeldenummer: 98113045.3
(22) Anmeldetag: 14.07.1998
(51) Int. Cl.: G01N 33/48, G01N 33/86

(54) **Verwendung von Annexinen als Lupus Antikoagulans Kontrolle oder Standard in Gerinnungstesten**
Use of annexine as lupus anticoagulation controle or standard in coagulation tests
Utilisation de l'annexine pour le controle des lupus anticoagulants or étalon dans l'essais de coagulation

(30) Priorität: 11.08.1997 DE 19734648
(43) Veröffentlichungstag der Anmeldung: 24.02.1999
(73) Patentinhaber: Dade Behring Marburg GmbH, 35001 Marburg (DE)
(72) Erfinder: Kraus, Michael, Dr., 35041 Marburg (DE)

(56) Entgegenhaltungen:
- WO-A-91/16453
- US-A- 4 877 741
- MCGLASSON ET AL : AM J CLIN PATHOL, Bd. 100, 1993, Seiten 576-8, XP000995688
- BRANDT J T ET AL: "LABORATORY IDENTIFICATION OF LUPUS ANTICOAGULANTS: RESULTS OF THE SECOND INTERNATIONAL WORKSHOP FOR IDENTIFICATION OF LUPUS ANTICOAGULANTS. ON BEHALF OF THE SUBCOMMITTEE ON LUPUS ANTICOAGULANTS/ANTIPHOSPHOLIPID ANTIBODIES OF THE ISTH" THROMBOSIS AND HAEMOSTASIS,DE,STUTTGART, Bd. 74, Nr. 6, 1. Dezember 1995 (1995-12-01), Seiten 1597-1603, XP000196768 ISSN: 0340-6245
- "Guidelines on testing for the lupus anticoagulant" J. CLINICAL PATHOLOGY, Bd. 44, 1991, Seiten 885-9, XP000995681
- BRANDT ET AL: THROMBOSIS AND HAEMOSTASIS, Bd. 74, Nr. 4, Oktober 1995 (1995-10), Seiten 1185-1190, XP000995683

## Beschreibung

Die Erfindung bezieht sich auf ein Verfahren zur Herstellung von Plasmen zur Anwendung als Kontrolle oder Standard in allen funktionellen Gerinnungstesten, die zum Nachweis eines Lupus Antikoagulans dienen.

Lupus Antikoagulantien sind Immunglobuline und gehören zum Formenkreis der worbenen Autoantikörper. Sie sind dadurch gekennzeichnet, daß sie gegen Phospholipide oder Phospholipid/Protein-Komplexe gerichtet sind und in gängigen Suchtesten der Gerinnung die Gerinnungszeit verlängern (siehe Triplett D., et al. Hematologic Pathology 1988; 2: 121-143). Diese Immunglobuline sind abzugrenzen von anderen, ebenfalls gegen Lipide, insbesondere Cardiolipine, gerichteten Autoantikörpem. Beide Gruppen werden klinisch dem Antiphospholipid Syndrom (APS) zugeordnet, das sich in Thrombosen und einer Häufung von Geburtskomplikationen (Fehlgeburten) manifestiert. Der Pathomechanismus der Lupus Antikoagulantien ist aus mehreren Gründen noch ungeklärt. Erstens ist die Spezifität der auftretenden Antikörper und somit der Wirkmechanismus individuel von Patient zu Patient verschieden. Zweitens variieren die Subklassen der Immunglobuline (IgM, IgG, IgA) und die Antikörpertiter. Drittens besteht zwischen der Bestimmung des Lupus Antikoagulans und der klinischen Manifestation ein Paradoxon: Eine Verlängerung der Gerinnungszeit in in vitro Testen, wie sie durch Lupus Antikoagulans hervorgerufen wird, deutet auf eine erhöhte Blutungsneigung, in vivo hingegen manifestiert sie sich in einer erhöhten Thromboseneigung.

Die Diagnose eines Lupus Antikoagulans beschränkt sich daher auf eine phänomenologische Beschreibung des Verhaltens einer Plasmaprobe in verschiedenen Gerinnungstesten entsprechend den Empfehlungen internationaler Komitees (Brandt, J.T. et al.,Thrombosis Haemostasis 1995; 74: 1185-1190.) Bei diesen Testen handelt es sich um die aktivierte partielle Thromboplastin-Zeit (APTT), die verdünnte Thromboplastin-Zeit (dPT) und die Russell's Viper Venom Zeit (RVVT). Eine Verlängerung der Gerinnungszeit wird in diesen Testen jedoch auch durch einen Faktorenmangel erhalten, weshalb im sog. Plasmatauschversuch die pathologische Probe mit normalem Plasma gemischt und in der Regel in der APTT bestimmt wird. Ein Faktorenmangel ist in der Regel bereits bei einer Substitution von 50% durch die Mischung mit dem normalen Plasma kompensiert, während in Anwesenheit eines Lupus Antikoagulans noch pathologische Ergebnisse erhalten werden. Weiterhin ist die Phospholipid-Abhängigkeit zu prüfen, die mit den gleichen Reagenzien, jedoch unterschiedlichen Konzentrationen an Phospholipiden durchgeführt wird. Abzugrenzen sind weiterhin Autoantikörper gegen einzelne Gerinnungsfaktoren, die ebenfalls nicht durch eine 1+1 Mischung mit einem normalen Plasma kompensiert werden. Diese Faktorenantikörper wirken in der Regel jedoch nur in einem der beiden Wege des Gerinnungssystems (insbesondere sog. Faktor VIII-Inhibitoren) und werden durch den Vergleich der verschiedenen Wege, d.h. durch den Vergleich der obengenannten Teste (APTT für den intrinsischen, PT für den extrinsischen Weg) erkannt.

Die Sensitivität der Reagenzien der oben genannten Teste auf Lupus Antikoagulans ist sehr unterschiedlich (Messmore, H., et al., Thrombosis and Hemostasis. 1994; 20: 79-94). Weiterhin verursachen Lupus Antikoagulantien nicht in allen Testen ein pathologisches Ergebnis, weshalb die Verwendung von mindestens zwei funktionellen Testen empfohlen wird (beispielsweie die APTT und die RVVT; siehe Brandt, J.T. et al., (1995), 74: 1185-1190). Zum Vergleich oder Austausch von Daten, z. B. bei klinischen Studien, wäre daher der Bezug auf einen Standard sinnvoll, ebenso wie eine regelmäßige Kontrolle der Testergebnisse, etwa zur Verfolgung einer Therapie. Die Verwendung von individuellen Plasmaspendern ist für die Herstellung eines solchen Standards oder einer Kontrolle jedoch aus Gründen der Ethik sowie der Heterogenität der Spezifität, der geringen Reproduzierbarkeit und der schlechten Stabilität von Lupus Antikoagulantien für die kommerzielle Nutzung ungeeignet. Es gibt daher zur Zeit weder eine eindeutige Bestimmung eines Lupus Antikoagulans noch eine Referenz zur Quantifizierung eines Lupus Antikoagulans.

Der Erfindung lag daher die Aufgabe zugrunde eine Verfahren zu finden, mit dem in einer Probe, z. B. Plasma, reproduzierbar und quantifizierbar ein Lupus Antikoagulans derart simuliert werden kann, daß dieses veränderte Plasma in allen gängigen funktionellen Gerinnungstesten pathologische Ergebnisse vergleichbar mit dem Vorliegen eines natürlichen Lupus Antikoagulans erzeugt und somit zur Quantifizierung der Wirkung (Standard) und als Kontrolle geeignet ist.

Ein wesentliches Charakteristikum des Lupus Antikoagulans ist die Abhängigkeit der Gerinnungs-verlängernden Eigenschaft von der Verfügbarkeit der Phospholipide. Eine Möglichkeit diese Verhalten zu simulieren wurde von Babcock und McGlasson (US 4,877,741) beschrieben. Sie verwenden einen Extrakt aus einer Spinne (Loxosceles reclusa), das ein Enzym mit einer Sphingomyelinase-D Aktivität enthält. Sie konnten zeigen (McGlasson, D.L. et al., Am. J. Clin. Pathol 1993; 100: 576-578), daß dieser Extrakt in der APTT bei verschiedenen Reagenzien zu einer Verlängerung der Gerinnungszeit führt und durch Zugabe von Phospholipiden neutralisiert werden kann. Diese Verlängerung ist jedoch nur schwach ausgeprägt. So betrug die erzielte Verlängerung maximal 63% des oberen Normbereichs (APTT-Reagenz von Pacific Hemostasis; Tabelle 2; MacGlasson, D.L. et al., Am. J. Clin. Pathol 1993; 100: 576-578). Dies ist zu gering um typische Verlängerungen von mehr als 100% zu erzeugen, wie sie bei hohem Lupus Antikoagulanz auftreten und daher für eine Standardisierung mit einem weiten Meßbereich nicht ausreichend.

Es wurde auch beschrieben, daß Annexine unter bestimmten Umständen zu einer Verlängerung der APTT führen können. Diese Familie von intrazellulären Proteinen umfaßt zur Zeit mindestens acht charakterisierte Proteine, die entsprechend der neuen Nomenklatur als Annexin I bis VIII bezeichnet werden (Römisch, J. et al., Biol. Chem. Hoppe Seyler 1990; 5: 383-388). Diese Proteine sind dadurch charakterisiert, daß sie entzündungsmodulierend wirken, indem sie bei Entzündungen aus Zellen freigesetzt an Membranoberflächen binden und dadurch die Bindung von Phospholipase A2, ein wichtiger Schritt zur Bildung von inflammatorisch wirksamen Arachidonsäure-Derivaten, inhibieren. Durch diese Kalzium-abhängige Bindung an Phospholipid-Oberflächen werden auch die an diesen Oberflächen ablaufenden Gerinnungsprozesse gestört, weshalb diese Proteine auch als "vaskuläres Antikoagulans" bezeichnet wurden. Typischerweise führt die Anwesenheit von Annexinen konzentrationsabhängig zu einer Verlängerung der APTT (Römisch et al. Thrombosis Research 1990; 60: 355-366).

Unbekannt war bisher jedoch, ob Annexine vergleichbar zu einem natürlichen Lupus Antikoagulans, alle Kriterien erfüllen können. Für die funktionelle Diagnostik eines Lupus Antikoaguans werden folgende Kriterien herangezogen (Brandt, J.T. et al.,Thrombosis Haemostasis 1995; 74:1185-1190):
1. Verlängerung der Gerinnungszeit über den Normalbereich hinaus in mindestens 2 Testen, insbesondere der APTT und der RVVT.
2. Nachweis, daß diese Verlängerung Phospholipid-abhängig ist und durch entweder Zugabe von Phospholipiden oder durch Verwendung der gleichen Reagenzien wie unter 1. nur mit höheren Phospholipid-Konzentrationen neutralisiert werden kann.
3. Abgrenzung von einem möglichen Faktorenmangel durch Plasmatauschversuche, wobei in einer 3+1, 1+1 und 1+3 Mischung mit einem normalen Plama in einem Gerinnungstest, bevorzugterweise der APTT, diese Verlängerung charakteristischerweise erst mit einer hohen Verdünnung (1+3) neutralisiert wird, während dies bei einem Faktorenmangel bereits früher deutlich wird.
4. Abgrenzung von einem erworbenen Inhibitor (Autoantikörper gegen einen Gerinnungsfaktor), insbesondere gegen Faktor IX oder VIII (erworbene Hämophilia A bzw. B) dadurch charakterisiert, daß dieser nur in einem der beiden Gerinnungswege (intrinischer bzw. extrinsischer Weg) wirkt, während ein Lupus Antikoagulans unspezifisch alle phospholipidabhängigen Stufen in Gerinnungstesten stört.

Überraschenderweise konnte nun gezeigt werden, daß durch die geeignete Verwendung von Annexin V eine Lupus Antikoagulans Standard- bzw. Kontrollpräparation hergestellt werden kann, die alle oben beschriebenen Kriterien erfüllt.

Beispiel 1 zeigt die Verlängerung der gängigen Suchteste der Gerinnung, der APTT, der PT und der RVVT bei Zugabe eines Annexins (Annexin V) zu einem Plasmapool normaler Blutspender (Standard Human Plasma). Bei einer Zugabe von 125 mg Annexin V/I wurden Verlängerungen der Gerinnungszeiten von 470%, 31% und 200% oberhalb des oberen Normbereichs der jeweiligen Reagenzien erzielt. Dies zeigt, daß gegenüber dem bisherigen Stand der Technik (MacGlasson DL et al., Am. J. Clin. Pathol 1993; 100: 576-578) deutlich stärkere Verlängerungen der Gerinnungszeiten und damit die Möglichkeit zur Herstellung eines Standards und damit des Erreichens eines weiteren Kalibrationsbereichs verbessert wurde. Bei der PT ist diese Verlängerung nicht so stark ausgeprägt. Die PT ist im Gegensatz zur APTT und RVVTnicht als Suchtest für Lupus Antikoagulantien empfohlen.

Damit zeigt Beispiel 1 die Erfüllung von Kriterium 1 der Lupus Diagnostik, d.h. ein pathologisches Ergebnis in mindestens 2 funktionellen Gerinnungstesten, insbesondere der APTT und der RVVT. Die beiden natürlichen Lupus Antikoagulans positiven Plasmen verhalten sich vergleichbar.

In Beispiel 1 wurden auch ein Plasma mit Faktorenmangel, bzw. ein Plasma mit einem erworbenen Inhibitor beispielsweise getestet. Der Mangel an Faktor VIII bzw. der erworbenen Inhibitor gegen Faktor VIII führt nur in der APTT zu pathologischen Ergebnissen und nicht wie das Annexin-haltige Plasma oder die natürlichen Lupus Antikoagulans positiven Plasmen auch in der PT bzw. der RVVT. Damit wird auch das Kriterium 4 der Lupus Antikoagulans Diagnostik erfüllt.

Beispiel 2 zeigt die Ergebnisse zur Prüfung der Phospholipid-Abhängigkeit in der APTT, der PT und der RVVT. Verwendet wurden Reagenzien mit einem gegenüber dem in Beispiel 1 verwendeten Reagenzien veränderten Phospholipid-Gehalt. Das Verhältnis aus der Gerinnungszeit bei niedriger Phospholipid-Konzentration gegenüber der bei hoher Phospholipid-Konzentration ist bei Plasmen mit Verdacht auf Lupus Antikoagulans gegenüber Normalplasmen erhöht. Dieses Verhalten ist nur bei den natürlichen und dem simulierten Lupus Antikoagulans haltigen Plasma in der geforderten APTT und RVVT gegeben. Auch die PT ist in diesem Fall pathologisch. Damit ist Kriterium 2 der Lupus Diagnostik erfüllt. Der Normalplasmapool, als auch das Faktorenmangel-Plasma zeigen sich unauffällig. Allein das Plasma mit einem erworbenen Faktorinhibitor bleibt in dem APTT basierenden Verfahren verlängert.

Beispiel 3 zeigt die Ergebnisse zur Prüfung auf Faktorenmangel durch Plasmatauschversuche in der APTT. Bei einer Mischung von 1+3 mit dem Normalplasmapool sind alle pathologischen Plasmen, als auch das simulierte Lupus Antikoagulans positive Plasma verlängert. Bei einer 1+1 Verdünnung wird der Mangel im Faktormangelplasma bereits ausreichend kompensiert. Die Gerinnungszeit ist im Normbereich. Annexin-haltige Plasmen, die natürlichen, Lupus Antikoagulans positiven Plasmen, als auch das Faktorinhibitor-haltige Plasma sind im Test noch pathologisch. Dies zeigt zum einen die Erfüllung des 3. und letzten Kriteriums für die Diagnose eines Lupus Antikoagulans und damit die Möglichkeit Annexine zur Simulation eines Lupus Antikoagulans zur Anwendung als Kalibratoren oder Kontrollen in funktionellen Gerinnungstesten einzusetzen.

Weiterhin zeigt Beispiel 3, daß zur Erfüllung des 3. Kriterium, dem Plasmatauschver such, die antikoagulatorisch wirksame Konzentration des Annexins in einer Kontrolle so eingestellt werden muß, daß in einer 1+3 Verdünnung mit einem Normalplasmapool in einer APTT noch ein pathologisches Ergebnis erhalten wird. Im unverdünnten Zustand entspricht dies in diesen Beispielen einem Zusatz von mindestens 7 mg/l Annexin V. Selbst bei dieser Mindestmenge wird im unverdünnten Plasma damit eine Verlängerung der Gerinnungszeit über dem oberen Normalbereich hinaus von 87% erreicht (siehe Beispiel 1, Tabelle 1, APTT wenig PL). Diese Anforderung ist durch bisher beschriebene Verfahren (McGlasson, D.L. et al., Am. J. Clin. Pathol 1993; 100: 576-578) nicht möglich gewesen und damit war mit diesem bisherigen Verfahren hergestellten Plasmen nicht möglich ein Lupus Antikoagulans ähnliches Verhalten im Plasmatauschversuch zu erzeugen. Das erfinderische Verfahren ist dem bisherigen Verfahren damit deutlich überlegen.

Neben den in den Beispielen gezeigten funktionellen Gerinnungstesten können die erfindungsgemäßen Kontroll- bzw. Kalibrator-Plasmen auch in anderen, dem Flachmann bekannten funktionellen Gerinnungstesten angewendet werden (Messmore, H. et al., Thrombosis Haemostasis 1994; 71: 220-224.; Brandt, J.T., Thrombosis Hae-. mostasis 1991; 66: 453-458; Amout, J. et al., Brit. J. Haematol. 1994; 87: 94-99; Rauch, J. et al., Thrombosis Haemostasis 1989; 62: 892-896).

In einem Kontrollplasma wird die Konzentration von Annexin V vorzugsweise so gewählt, daß selbst bei einer Verdünnung dieses Kontrollplasmas 1+3 mit einem Normalplasmapool noch eine APTT oberhalb des Normbereichs erhalten wird. Bei Abstimmung der Kontrolle auf andere Gerinnungsteste können diese Grenzkonzentrationen reagenz- und testspezifisch voneinander abweichen.

Für Kalibratorplasmen wird die Konzentration von Annexin V idealerweise so gewählt, daß die damit erhaltenen Gerinnungszeiten oberhalb des Normbereichs des jeweiligen Tests und verwendeten Reagenzes liegen.

Aus der Verwendung von definierten Mengen von Annexin V zur Erzeugung einer Verlängerung der Gerinnungszeit ergibt sich die Möglichkeit zur Kalibration von Antikörper Titern von Lupus Antikoagulans positiven Plasmen beispielsweise in mg/l Annexin-Äquivalent.

Die nachfolgenden Beispiele erläutern die Erfindung.

Wenn nicht anders angegeben wurden im folgenden für die Beispiele Reagenzien und Plasmen der Fa. Behringwerke verwendet.

### Beispiel 1

Verhalten eines Annexin-haltigen Plasmas in Suchtesten der Gerinnung im Vergleich zu verschiedenen normalen und pathologischen Plasmen.

Ein Normalplasmapool (Standardhumanplasma) wurde mit 4 bis 500 mg/l rekombinanten Annexin V (hergestellt nach EP 0 271 885) versetzt und an einem Gerinnungsautomaten (Fa. Amelung, Deutschland) die Gerinnungszeiten in der APTT (Reagenz: Pathromtin SLA bzw Pathromtin SL mit 10-fach höherer Phospholipid-Konzentration als in Pathromtin SLA), der PT (Reagenz: Thromborel S) und der RVVT (Reagenz: LA Screen; Hersteller: Gradipore, Australien) bestimmt. Tabelle 1 zeigt die Verlängerung der gängigen Suchteste der Gerinnung, der APTT, der PT und der RVVT bei Zugabe eines Annexins (Annexin V) zu einem Normalplasmapool. Bei einer Zugabe von 125 mg Annexin V/I wurden die Gerinnungszeiten um > 400%, 31% und 200% oberhalb des oberen Normbereichs der jeweiligen Reagenzien (APTT: 38 sec bzw. 49 sec; PT: 13 sec; RVVT: 45 sec; gemäß Angaben der Hersteller) verlängert.

**Tabelle 1**

| Einfluß von annexi V in einem Normalplasmapool (NPP) auf die Suchteste der Gerinnung, APTT, PT und RVVT. | | | | |
|---|---|---|---|---|
| Gehalt von Annexin V in NPP (in mg/l) | APTT [sec] viel PL | APTT [sec] wenig PL | PT [sec] | RVVT [sec] |
| 0 | 38,0 | 45,5 | 13,0 | 38,8 |
| 3,8 | 37,6 | 59,1 | | |
| 7,3 | 38,8 | 91,6 | | |
| 15,5 | 40,1 | 149,3 | | |
| 31,3 | 45,0 | 186,3 | | |
| 62,5 | 81,7 | 211,6 | 15,0 | 122,6 |
| 125 | 215,9 | 262,5 | 17,0 | 135,1 |
| 250 | 233,5 | 270,0 | 21,2 | 138,4 |
| 500 | 320,6 | 316,0 | 34,5 | 152,8 |

Weiterhin wurde mit diesen Suchtesten die Gerinnungszeiten von folgenden pathologischen Plasmen ermittelt:
- Faktor VIII-Mangelplasma (Prod. Nr. OTXW) zur Simulation eines Faktorenmangels;
- ein Citrat-Plasma eines Patienten mit erworbenen Faktor VIII-Inhibitor (Fa. George King, USA);
- 2 Citrat-Plasmen von Patienten mit nachgewiesenem Lupus Antikoagulans (Fa. Trina, Schweiz).

Tabelle 2 zeigt die Ergebnisse der Bestimmung der APTT, der PT und der RVVT in den verschiedenen Plasmen. Der Normalplasmapool liegt innerhalb des Normbereichs aller 3 Teste. Das Faktor VIII-Mangelplasma hingegen ist in der APTT auffällig, ebenso wie das Plasma mit einem erworbenen Faktor VIII-Inhibitor. Beide Plasmen sind in der PT nur schwach pathologisch, in der RVVT hingegen unauffällig. Im Gegensatz dazu sind die beiden natürlichen Lupus Antikoagulans positiven Plasmen in allen Testen pathologisch, wenngleich in unterschiedlich starker Ausprägung. Diese unspezifische Verlängerung der Gerinnungszeiten in den Suchtesten der Gerinnung wird in gleicher Weise durch den Zusatz von Annexin zu einem Normalplasmapool erzeugt (in Tabelle 2 wurden die Ergebnisse aus Tabelle 1 mit einem Zusatz von 125 mg/l bzw. 62,5 mg/l Annexin übernommen).

### Tabelle 2

Verhalten von einem Normalplasmapool (NPP), einem Plasma mit Faktorenmangel (FVIII-MP) bzw. Inhibitor gegen Faktor VIII (Anti-FVIII-P), natürlichen Lupus Antikoagulans positiven Plasmen (LA 1; LA 2) und einem simulierten Lupus Antikoagulans (NPP+Annexin) in Suchtesten der Gerinnung, APTT, PT und RVVT. Angegeben ist weiterhin der Normbereich dieser Teste bei den verwendeten Reagenzien.

**Tabelle 2**

| Plasmaprobe | APTT [sec] | PT [sec] | RVVT [sec] |
|---|---|---|---|
| NPP | 38,0 | 13,0 | 38,8 |
| FVIII-MP | 124,3 | 14,5 | 44,5 |
| Anti-FVIII-P | 133,6 | 13,5 | 38,0 |
| LA 1 | 56,5 | 14,5 | 136,3 |
| LA 2 | 55,4 | 14,5 | 133,3 |
| NPP + 63 mg/l Annexin | 81,7 | 15,0 | 122,6 |
| Normbereich | 26 - 38 sec | 10 - 13 sec | 31 - 45 sec |

### Beispiel 2

Phospholipidabhängigkeit des Verhaltens eines Annexin-haltigen Plasmas in Suchtesten der Gerinnung im Vergleich zu verschiedenen normalen und pathologischen Plasmen.

Zur Prüfung der Phospholipid-Abhängigkeit wurde die APTT, PT und RVVT der in Beispiel 1 genannten pathologischen Plasmen, sowie des Normalplasmapools und einer simulierten Lupus Antikoagulans Kontrolle, bestehend aus Normalplasmapool und 125 bzw. 63 mg/l Annexin V, mit Reagenzien bestimmt, die gegenüber Beispiel 1 einen anderen Phospholipid-Gehalt aufweisen. Dies war für die APTT das Reagenz Pathromtin SLA mit einer gegenüber Beispiel 1 um 90% verringerten Phospholipid-Konzentration des Pathromtin SL. Für die PT-Bestimmung wurde nach Brauer et al. (J Clin Chem Clin Biochem 1990; 28: 701) Thromborel S 1:50 mit 12,5 mM Kalziumchlorid-Lösung verdünnt. Im Gegensatz dazu wird für die RVVT bereits in der Routine ein Lupus sensitives Reagenz, d.h. mit wenig Phospholipiden verwendet. Zur Gegenkontrolle wurde daher in diesem Beispiel mit LA-Confirm ein Reagenz mit höherer Phospholipid-Konzentration eingesetzt. Die Ergebnisse sind in Tabelle 3 aufgeführt.

### Tabelle 3

Verhalten von einem Normalplasmapool (NPP), einem Plasma mit Faktorenmangel (FVIII-MP) bzw. Inhibitor gegen Faktor VIII (Anti-FVIII-P), natürlichen Lupus Antikoagulans positiven Plasmen (LA 1; LA 2) und einem simulierten Lupus Antikoagulans (NPP+Annexin) in Suchtesten der Gerinnung, APTT, PT und RVVT, bei gegenüber Beispiel 1 verändertem Phospholipid-Gehalt (PL).

**Tabelle 3**

| | | | |
|---|---|---|---|
| Verhalten von einem Normalplasmapool (NPP), einem Plasma mit Faktorenmangel (FVIII-MP) bzw. Inhibitor gegen Faktor VIII (Anti-FVIII-P), natürlichen Lupus Antikoagulans positiven Plasmen (LA 1; LA 2) und einem simulierten Lupus Antikoagulans (NPP+Annexin) in Suchtesten der Gerinnung, APTT, PT und RVVT, bei gegenüber Beispiel 1 verändertem Phospholipid-Gehalt (PL). | | | |

| Plasmaprobe | APTT [sec] PL erniedrigt | PT [sec] PL erniedrigt | RVVT [sec] PL erhöht |
|---|---|---|---|
| NPP | 45,5 | 21,5 | 34,0 |
| FVIII-MP | 130,6 | 27,6 | 37,0 |
| Anti-FVIII-P | 178,2 | 24,2 | 35,8 |
| LA 1 | 162,7 | 44,8 | 45,5 |
| LA 2 | 156,8 | 39,7 | 42,0 |
| NPP + 63 mg/l Annexin | 211,6 | 54,0 | 34,7 |
| Normbereich | 32 - 49 sec | 16 - 27 sec | 34 - 51 sec |

Da in Anwesenheit von viel Phospholipiden die Störung der Gerinnung durch Lupus Antikoagulans verringert ist, wurden aus den in Tabellen 2 (Beispiel 1) und 3 erhaltenen Gerinnungszeiten der Quotient aus der Gerinnungszeit in Anwesenheit von wenig zu der bei Anwesenheit von viel Phospholipiden gebildet (siehe Tabelle 4). Eine Verkürzung der Gerinnungszeit auf Grund der Neutralisierung der inhibitorischen Wirkung eines Lupus Antikoagulans drückt sich in einem erhöhten Quotienten aus. Die für diese Reagenzien ermittelten zulässigen Höchstgrenzen, unterhalb derer kein Verdacht auf ein Lupus Antikoagulans besteht, sind in Tabelle 4 für die hier verwendeten Reagenzien aufgeführt.

Es zeigt sich, daß der Normalplasmapool, auch bei Vorliegen eines Faktorenmangels, Quotienten in allen Testen im Normbereich liefert. Das Plasma mit einem spezifischen Inhibitor hingegen ist nur in der APTT Ratio noch pathologisch. Für die natürlichen Lupus Antikoagulans haltigen Plasmen, als auch für das erfindungsgemäße, simulierte Lupus Antikoagulans Plasma, wurde in allen 3 Testen eine unspezifische Reaktion mit Phospholipiden durch deutlich gegenüber dem Normbereich erhöhten Quotienten erhalten.

**Tabelle 4**

| | | | |
|---|---|---|---|
| Berechnung der Phospholipid-Abhängigkeit von einem Normalplasmapool (NPP), einem Plasma mit Faktorenmangel (FVIII-MP) bzw. Inhibitor gegen Faktor VIII (Anti-FVIII-P), natürlichen Lupus Antikoagulans positiven Plasmen (LA 1; LA 2) und einem simulierten Lupus Antikoagulans (NPP+Annexin) in Suchtesten der Gerinnung, APTT, PT und RVVT, durch Bildung des Quotienten aus der Gerinnungszeit in Anwesenheit von wenig zu der bei viel Phospholipiden. Weiterhin ist der zulässige Grenzwert des Quotienten angegeben, oberhalb dem ein Verdacht auf das Vorliegen eines Lupus Antikoagulans besteht. | | | |

| Plasmaprobe | APTT-Ratio | PT-Ratio | RVVT-Ratio |
|---|---|---|---|
| NPP | 1,2 | 1,7 | 1,1 |
| FVIII-MP | 1,1 | 1,9 | 1,2 |
| Anti-FVIII-P | 1,3 | 1,8 | 1,1 |
| LA 1 | 2,9 | 3,1 | 3,0 |
| LA 2 | 2,8 | 2,7 | 3,2 |
| NPP + 63 mg/l Annexin | 2,6 | 3,6 | 3,5 |
| oberer Grenzwert | 1,2 | 2,1 | 1,3 |

### Beispiel 3

Verhalten eines Annexin-haltigen Plasmas und verschiedener pathologischer Plasmen im Plasmatauschversuch.

Der Plasmatauschversuch wurde in der APTT unter Verwendung von Pathromtin SL durchgeführt. Hierzu wurden die in Beispiel 1 genannten pathologischen Plasmen als auch das simulierte Lupus Antikoagulans positive Plasma 3+1, 1+1 und 1+3 mit einem Normalplasmapool gemischt und die APTT bestimmt. Die Ergebnisse sind in Tabelle 5 aufgeführt.

**Tabelle 5**

| | | | |
|---|---|---|---|
| Verhalten eines Plasmas mit Faktorenmangel (FVIII-MP) bzw. Inhibitors gegen Faktor VIII (Anti-FVIII-P), natürlichen Lupus Antikoagulans positiven Plasmen (LA 1; LA 2) und einem simulierten Lupus Antikoagulans (NPP+Annexin) im Plasmatauschversuch. Angegeben ist die APTT (mittels Pathromtin SLA) in sec bei verschiedenen Mischungen (3+1, 1+1, 1+3; Probe + NPP) mit einem Normalplasmapool (NPP). | | | |

| Plasmaprobe | 3+1 | 1+1 | 1+3 |
|---|---|---|---|
| FVIII-MP | 56,9 | 48,9 | 46,5 |
| Anti-FVIII-P | 125,4 | 88,5 | 64,8 |
| LA 1 | 142,3 | 123,7 | 105,5 |
| LA 2 | 137,7 | 118,0 | 101,6 |
| NPP+ 63 mg/l Annexin | 184,3 | 171,2 | 139,6 |
| NPP+ 7,5 mg/l Annexin | 81,6 | 65,5 | 56,6 |

Bei einer Mischung von 1 Teil Normalplasmapool mit 3 Teilen der Plasmen ("3+1" in Tabelle 5) reagieren alle pathologischen Plasmen, als auch das simulierte Lupus Antikoagulans positive Plasma pathologisch. Bei einer 1+1 Verdünnung wird der Mangel im Faktormangelplasma bereits vollständig kompensiert (obere Grenze des Normbereich 50 sec). Die beiden natürlichen Lupus Antikoagulans positiven Plasmen, die Annexin-haltigen Plasmen, als auch das Faktorinhibitor haltige Plasma sind im Test auch bei höherer Verdünnung (1+3) noch hoch pathologisch.

## Patentansprüche

1. Kontroll- und/oder Kalibrationsplasma zur Verwendung in Gerinnungstesten, **dadurch gekennzeichnet, daß** es Annexin V und Plasma enthält und daß durch Zugabe von Annexin V zum Plasma ein Lupus Antikoagulans initiiert wird.

2. Kontroll- und/oder Kalibrationsplasma nach Anspruch 1, **dadurch gekennzeichnet, daß** das verwendete Annexin aus natürlichen Quellen isoliert wurde oder rekombinanten Ursprungs ist.

3. Plasma nach Anspruch 1, **dadurch gekennzeichnet, daß** es sich um Plasma humanen und/oder nicht-humanen Ursprungs, bevorzugterweise humanen Ursprungs handelt.

4. Plasma nach Anspruch 1 oder 3, **dadurch gekennzeichnet, daß** es sich um Plasmen von normalen Tieren und/oder Spendern handelt.

5. Plasma nach einem der Ansprüche 1, 3 oder 4, **dadurch gekennzeichnet, daß** diese Plasmen einen Mangel an Faktoren des humoralen Systems aufweisen, der natürlichen Ursprungs ist oder künstlich erzeugt wurde.

6. Plasma nach Anspruch 5, **dadurch gekennzeichnet, daß** der Mangel an Faktoren des humoralen Systems künstlich durch Affnitäts- und/oder Immunchromatographie erzeugt wurde.

7. Plasma nach einem der Ansprüche 1 oder 3-6, **dadurch gekennzeichnet, daß** zur Stabilisierung oder zur Vermeidung unerwünschter Nebenreaktionen als Zusätze Zucker, Aminosäuren, Alkohole, natürliche und synthetische Inhibitoren von Proteasen, sowie Chelatbildner, Antioxidantien, Antibiotika und Azide zugesetzt sind.

8. Gerinnungstest unter Verwendung eines Kontroll- und/oder Kalibrationsplasma nach einem der Ansprüche 1 oder 2.

9. Gerinnungstest zur Bestimmung von Lupus Antikoagulans nach Anspruch 8, **dadurch gekennzeichnet, daß** es sich um einen Suchtest wie der aktivierten partiellen Thromboplastinzeit (APTT), der Thromboplastinzeit (PT), der Russell's Viper Venom Zeit (RVVT) oder der Kaolin Gerinnungszeit (KCT) handelt.

10. Gerinnungstest zur Bestimmung von Lupus Antikoagulans nach Anspruch 8, **dadurch gekennzeichnet, daß** es sich um Gerinnungsteste handelt, in denen eine phospholipidabhängige Umsetzung von Gerinnungsfaktoren erfolgt, wie beispielsweise durch Zusatz einzelner, aktivierter Gerinnungsfaktoren, wie Faktor VIIa, Faktor IXa, Faktor Xa, Thrombin, Protein Ca.

11. Gerinnungstest zur Bestimmung von Lupus Antikoagulans nach mindestens einem der Ansprüche 8-10, **dadurch gekennzeichnet, daß** der Nachweis der Gerinnungsaktivität durch Verfahren wie dem mechanischen, elektrischen oder optischen Nachweis einer Gerinnselbildung oder dem Nachweis der Aktivierung bestimmter Proteasen der Gerinnung durch Zusatz geeigneter chromogener Substrate durchgeführt wird.

12. Verfahren zur Prüfung der Phospholipid-Abhängigkeit unter Verwendung des Kontroll- und/oder Kalibrationsplasma nach Anspruch 1 oder 2, **dadurch gekennzeichnet, daß** Phospholipide zum Testansatz über das Reagenz, durch Zusatz der Probe oder getrennten Zusatz mit oder ohne Vorinkubation der Probe mit dem Phospholipid, zugegeben werden.

13. Verfahren nach Anspruch 12, **dadurch gekennzeichnet, daß** als Phospholipide synthetische oder natürliche Phospholipide, beispielsweise aus Plazenta, Plättchen, Pflanzenextrakten in undefinierter oder in definierter Organisation, beispielsweise in hexagonaler Anordung, uni- oder multilamellar verwendet werden.

## Claims

1. A control and/or calibration plasma for use in clotting tests, characterized that it comprises annexin V and plasma and in that a lupus anticoagulant is initiated by addition of annexin V to the plasma.

2. A control and/or calibration plasma as claimed in claim 1, **characterized in that** annexin used has been isolated from natural sources or is of recombinant origin.

3. A plasma as claimed in claim 1, **characterized in that** it is plasma of human and/or nonhuman origin, preferably human origin.

4. A plasma as set forth in claim 1 or 3, **characterized in that** it is plasma from normal animals and/or donors.

5. A plasma as set forth in any of claims 1, 3 and 4, **characterized in that** these plasmas has a deficiency of factors of the humoral system, which is of natural origin or was produced artificially.

6. A plasma as claimed in claim 5, **characterized in that** the deficiency of factors of the humoral system was produced artificially by affinity and/or immunochromatography.

7. A plasma as set forth in any of claims 1 or 3-6, wherein, for stabilization or for the avoidance of undesired side reactions, sugars, amino acids, alcohols, natural and synthetic inhibitors of proteases, and chelating agents, antioxidants, antibiotics and azides, are added as additives.

8. A clotting test using a control and/or calibration plasma as set forth in either of claims 1 and 2.

9. A clotting test for the determination of lupus anticoagulant as set forth in claim 8, **characterized in that** it comprises a diagnostic test such as the activated partial thromboplastin time (APTT), the partial thromboplastin time (PT), the Russell's viper venom time (RVVT) or the kaolin clotting time (KCT).

10. A clotting test for the determination of lupus anticoagulant as set forth in claim 8, **characterized in that** it comprises a clotting test in which a phospholipid-dependent reaction of clotting factors takes place, such as, for example, as a result of addition of individual, activated clotting factors, such as Factor VIIa, Factor IXa, Factor Xa, thrombin, protein Ca.

11. A clotting test for the determination of lupus anticoagulant as set forth in at least one of claims 8-10, **characterized in that** it comprises carrying out the detection of the clotting activity by processes such as the mechanical, electrical or optical detection of clot formation or the detection of the activation of certain proteases of clotting by addition of suitable chromogenic substrates.

12. A method of testing the phospholipid dependence using the control and/or calibration plasma as set forth in claim 1 or 2, **characterized in that** phosholipids are added to the test batch via the reagent, by addition of the sample or separate addition with or without preincubation of the sample with the phospholipid.

13. A method as set forth in claim 12, **characterized in that**, as phospholipids, synthetic or natural phospholipids, for example from placenta, platelets, plant extracts, can be used in uni- or multilamellar form in undefined or in defined organization, for example in hexagonal arrangement.

## Revendications

1. Plasma témoin et/ou étalon pour utilisation dans les tests de coagulation, **caractérisé en ce qu'**il contient de l'annexine V et du plasma, et **en ce qu'**un anticoagulant lupique est initié par l'addition d'annexine V au plasma.

2. Plasma temoin et/ou étalon selon la revendication 1, **caractérisé en ce que** l'annexine utilisée a été isolée à partir de sources naturelles ou est d'origine recombinante.

3. Plasma selon la revendication 1, **caractérisé en ce qu'**il s'agit d'un plasma d'origine humaine et/ou non humaine, de préférence d'origine humaine.

4. Plasma selon une des revendications 1 ou 3, **caractérisé en ce qu'**il s'agit de plasmas d'animaux et/ou de donneurs normaux.

5. Plasma selon une des revendications 1, 3 ou 4, **caractérisé en ce que** ces plasmas présentent un déficit en facteurs du système humoral, qui est d'origine naturelle ou a été produit artificiellement.

6. Plasma selon la revendication 5, **caractérisé en ce que** le déficit en facteurs du système humoral a été produit artificiellement par chromatographie d'affinité et/ou immunochromatographie.

7. Plasma selon un des revendications 1 ou 3 à 6, **caractérisé en ce que** des sucres, des acides aminés, des alcools, des inhibiteurs naturels et synthétiques des protéases, ainsi que des agents de chélation, des antioxydants, des antibiotiques et des azides sont ajoutés comme additifs à des fins de stabilisation ou pour éviter des réactions secondaires indésirables.

8. Test de coagulation utilisant un plasma témoin et/ou étalon selon une des revendications 1 ou 2.

9. Test de coagulation pour la détermination d'un anticoagulant lupique selon la revendication 8, **caractérisé en ce qu'**il s'agit d'un test experimental tel que le temps de thromboplastine partielle activée (APTT), le temps de thromboplastine (PT), le temps de venin de vipère Russell (RVVT) ou le temps de coagulation de Kaolin (TCK)).

10. Test de coagulation pour la détermination d'un anticoagulant lupique selon la revendication 8, **caractérisé en ce qu'**il s'agit de tests de coagulation dans lesquels il se produit une transformation en fonction des phospholipides des facteurs de coagulation, par exemple par addition de facteurs de coagulation activés individuels, comme le facteur VIIa, le facteur IXa, le facteur Xa, la thrombine, la proteine Ca.

11. Test de coagulation pour la détermination d'un anticoagulant lupique selon au moins une des revendications 8 à 10, **caractérisé en ce que** l'identification de l'activité coagulante est effectuée par des procédés tels que l'identification mécanique, électrique ou optique de la formation d'un coagulum ou l'identification de l'activation de certaines protéases de la coagulation par l'addition de substrats chromogènes appropriés

12. Procédé de vérification de la dépendance aux phospholipides en utilisant le plasma témoin et/ou d'étalonnage selon les revendications 1 ou 2, **caractérisé en ce que** les phospholipides sont ajoutés à la préparation d'essai par l'intermédiaire du réactif, par addition de l'échantillon ou addition séparée avec ou sans préincubation de l'échantillon avec le phospholipide.

13. Procédé selon la revendication 12, **caractérisé en ce que** en tant que phospholipides, sont utilisés des phospholipides synthétiques ou naturels, par exemple de placenta, de plaquettes, d'extraits végétaux à structure définie ou non définie, par exemple en disposition hexagonale, unilamellaire ou multilamellaire.
